# EUROPEAN PATENT APPLICATION

(11) **EP 4 818 308 A1**
(43) Date of publication of application: **07.10.2026**
(21) Application number: 24898262.1
(22) Date of filing: 02.12.2024
(51) Int. Cl.: C12N 1/20, A23K 10/16, A23K 50/10, C12R 1/01

(54) **COMPOSITION FOR REDUCING METHANE EMISSION FROM RUMINANT ANIMALS**

(30) Priority: 30.11.2023 KR 20230171473; 29.11.2024 KR 20240174619
(71) Applicant: SYNB Co., Ltd., Gyeongsangnam-do 52828 (KR); Lee, Sang Suk, Jeollanam-do 57935 (KR)
(72) Inventor: KIM, Seon Won, Sejong 30062 (KR); KWON, Moon Hyuk, Jinju-si, Gyeonggi-do 52831 (KR); KANG, Min Kyoung, Jeju-si, Jeju-do 63292 (KR); YOON, Sang Hwal, Jinju-si, Gyeongsangnam-do 52857 (KR); REYANTHONY, Sanjorjo, Jinju-si, Gyeongsangnam-do 52828 (KR); TENZIN, Tseten, Jinju-si, Gyeongsangnam-do 52828 (KR); LEE, Sang Suk, Suncheon-si, Jeollanam-do 57935 (KR); BAIK, Keun Sik, Gwangju 61073 (KR)
(74) Representative: Isarpatent
(86) International application number: PCT/KR2024/019504
(87) International publication number: WO 2025/116670

(57) **Abstract**

The present invention relates to a composition which, by comprising a methanotrophic bacterium, can effectively reduce methane emission from a ruminant animal and can improve the feed efficiency, fermentation efficiency, and the like of a ruminant animal, and to a method for reducing methane emission from a ruminant animal. In particular, the present invention relates to a composition for reducing methane emission from a ruminant animal, the composition comprising a methanotrophic bacterium, to a feed composition for a ruminant animal, and to a feed additive composition for a ruminant animal.

## Description

### TECHNICAL FIELD

The present invention relates to a composition for reducing methane emission from a ruminant animal.

### BACKGROUND ART

Methane is a gas having a greenhouse effect approximately 80 times greater than that of carbon dioxide and is a major cause of global warming. However, since the lifetime of a methane molecule is more than 10 times shorter than that of carbon dioxide, it has been reported that reducing methane generation is more immediate and effective in responding to global warming than reducing carbon dioxide. Accordingly, through the Global Methane Pledge in 2021, the world declared to reduce methane emission by 30% by 2030 compared to 2018 levels. Therefore, reducing methane generation is very important for a globally sustainable industry. As for the major sources of methane generation, more than approximately 37% of global methane emission originates from ruminant animals such as cattle. For an eco-friendly livestock industry, the development of a composition capable of reducing methane generated from such industries is important.

Conventionally, methane reduction in ruminant animals has been carried out by adding a compound that inhibits the growth of methane-producing microorganisms. This is undesirable in that it inhibits the growth of methanogens, which are important constituent microorganisms of rumen fermentation, thereby causing a reduction in rumen fermentation efficiency and feed efficiency.

### DISCLOSURE OF THE INVENTION

### TECHNICAL PROBLEM

An object of the present invention is to provide a composition and a method capable of effectively reducing methane emission in a ruminant animal.

### TECHNICAL SOLUTION

1. A composition for reducing methane emission from a ruminant animal, comprising a methanotrophic bacterium.
2. The composition for reducing methane emission from a ruminant animal according to item 1 above, wherein the methanotrophic bacterium is derived from the rumen of a ruminant animal.
3. The composition for reducing methane emission from a ruminant animal according to item 1 above, wherein the methanotrophic bacterium is a bacterium of the genus Methylocystis, the genus Methylomonas, the genus Methylococcus, the genus Methylosinus, or the genus Methylomicrobium.
4. The composition for reducing methane emission from a ruminant animal according to item 1 above, further comprising a methylotrophic bacterium.
5. The composition for reducing methane emission from a ruminant animal according to item 4 above, wherein the methylotrophic bacterium is derived from the rumen of a ruminant animal.
6. The composition for reducing methane emission from a ruminant animal according to item 4 above, wherein the methylotrophic bacterium is a bacterium of the genus Methylobacterium.
7. The composition for reducing methane emission from a ruminant animal according to item 4 above, wherein the methylotrophic bacterium is Methylobacterium organophilum.
8. The composition for reducing methane emission from a ruminant animal according to item 1 above, wherein the ruminant animal is a cow, a giraffe, a deer, a sheep, or a camel.
9. A method for reducing methane emission from a ruminant animal, comprising feeding the composition according to any one of items 1 to 8 above to a ruminant animal.
10. A feed composition for a ruminant animal, comprising a methanotrophic bacterium.
11. The feed composition for a ruminant animal according to item 10 above, wherein the methanotrophic bacterium is derived from the rumen of a ruminant animal.
12. The feed composition for a ruminant animal according to item 10 above, wherein the methanotrophic bacterium is a bacterium of the genus Methylocystis, the genus Methylomonas, the genus Methylococcus, the genus Methylosinus, or the genus Methylomicrobium.
13. The feed composition for a ruminant animal according to item 10 above, further comprising a methylotrophic bacterium.
14. The feed composition for a ruminant animal according to item 13 above, wherein the methylotrophic bacterium is derived from the rumen of a ruminant animal.
15. The feed composition for a ruminant animal according to item 13 above, wherein the methylotrophic bacterium is a bacterium of the genus Methylobacterium.
16. The feed composition for a ruminant animal according to item 13 above, wherein the methylotrophic bacterium is Methylobacterium organophilum.
17. A feed additive composition for a ruminant animal, comprising a methanotrophic bacterium.
18. The feed additive composition for a ruminant animal according to item 17 above, wherein the methanotrophic bacterium is derived from the rumen of a ruminant animal.
19. The feed additive composition for a ruminant animal according to item 17 above, wherein the methanotrophic bacterium is a bacterium of the genus Methylocystis, the genus Methylomonas, the genus Methylococcus, the genus Methylosinus, or the genus Methylomicrobium.
20. The feed additive composition for a ruminant animal according to item 17 above, further comprising a methylotrophic bacterium.
21. The feed additive composition for a ruminant animal according to item 20 above, wherein the methylotrophic bacterium is derived from the rumen of a ruminant animal.
22. The feed additive composition for a ruminant animal according to item 20 above, wherein the methylotrophic bacterium is a bacterium of the genus Methylobacterium.
23. The feed additive composition for a ruminant animal according to item 20 above, wherein the methylotrophic bacterium is Methylobacterium organophilum.

### ADVANTAGEOUS EFFECTS

The present invention has an excellent methane emission reduction effect in ruminant animals.

The present invention is capable of improving feed efficiency in ruminant animals.

The present invention is capable of improving fermentation efficiency in ruminant animals.

### BRIEF DESCRIPTION OF THE DRAWINGS

FIG. 1 shows growth tests of three isolates NC52PC, NC75PC, and NC77PC. Growth curves (OD600) of the three isolates from 0 hours to 36 hours at 30°C (A) and 39°C (B).
FIG. 2 shows SEM images of NC52PC cells showing two morphologically distinct cell types.
FIG. 3 shows: A) a circular chromosomal map of the entire genome of Methylocystis sp. and two plasmids, including a circular chromosomal map showing genome comparison with closely related species, in which the innermost ring represents GC skew (green -, purple +) and GC content (black), and the rings and colors in the legend represent closely related strains used for comparison with Methylocystis sp. of NC52PC; and B) a circular chromosomal map comparing the entire genome of Methylobacterium sp. of NC52PC with closely related species.
FIG. 4 shows the pH and headspace gas volume (A and B) of the control and NC52PC-inoculated samples after 0, 12, and 24 hours, and the digestibility and methane production (C and D) between the control and NC52PC-inoculated samples after 12 and 24 hours. "ns" indicates not significant (p-value > 0.05), and an asterisk indicates a significant difference (p-value < 0.05).
FIG. 5 shows taxonomic analysis of in vitro rumen fermentation samples. Relative abundance at the phylum level for bacteria (A), archaea (B), and fungi (C). Relative abundance at the genus level for bacteria (D) and archaea (E). pmoA copy numbers for the methanotroph population between the control and NC52PC samples (F).
FIG. 6 shows alpha diversity of in vitro rumen fermentation samples. A) Shannon index for bacteria, archaea, and fungi. B) Chao 1 index for bacteria, archaea, and fungi.
FIG. 7 shows animal performance such as total body weight gain (A) and feed conversion ratio (B) among three animal groups: Control, LOW, and HIGH. "ns" indicates not significant (p-value > 0.05).
FIG. 8 shows the effects of low-concentration and high-concentration methanotroph-based probiotics (NC52PC) on methane emission (A), methane yield (B), and methane intensity (C) during three replicated in vivo test periods.

### MODE FOR CARRYING OUT THE INVENTION

Hereinafter, the present invention will be described in detail.

The present invention relates to a composition for reducing methane emission from a ruminant animal, comprising a methanotrophic bacterium.

The composition of the present invention is fed to a ruminant animal and metabolizes methane generated in the rumen of the ruminant animal, thereby reducing methane emission.

The ruminant animal may be, for example, a cow, a giraffe, a deer, a sheep, a camel, or the like. The ruminant animal may be a cow. The ruminant animal may be of the same species as the ruminant animal to which the composition is to be administered.

The methanotrophic bacterium may be one capable of growing and being active in the rumen.

For example, the methanotrophic bacterium may exhibit active growth even at 39°C. This means that growth inhibition is not significant compared with growth at 30°C, wherein the degree of growth inhibition may be 80% or less, 50% or less, 40% or less, 30% or less, 20% or less, or 10% or less.

For example, the methanotrophic bacterium may exhibit active growth in rumen fluid. This means that growth inhibition is not significant compared with NMS medium, which is a basic growth medium, wherein the degree of growth inhibition may be 80% or less, 50% or less, 40% or less, 30% or less, 20% or less, or 10% or less.

For example, the methanotrophic bacterium may be capable of growing even under anaerobic or partially aerobic conditions. For example, the methanotrophic bacterium may have an ability to utilize an alternative electron acceptor in place of oxygen under anaerobic conditions.

The methanotrophic bacterium (methanotroph) may be derived from the rumen of a ruminant animal.

The methanotrophic bacterium may be an aerobic or anaerobic strain.

The methanotrophic bacterium may be, for example, a strain of the genus Methylocystis, the genus Methylomonas, the genus Methylococcus, the genus Methylosinus, or the genus Methylomicrobium. For example, the methanotrophic bacterium may be Methylocystis parvus, Methylocystis rosea, Methylocystis ecosymbiosis, or Methylocystis echinoides.

The composition of the present invention may further comprise a methylotrophic bacterium.

The methylotrophic bacterium may promote growth of the methanotrophic bacterium, thereby further enhancing the methane emission-reducing effect.

The methylotrophic bacterium may be derived from the rumen of a ruminant animal. In such a case, the ruminant animal from which the methylotrophic bacterium is derived may be an animal of the same species as the ruminant animal from which the methanotrophic bacterium is derived.

The methylotrophic bacterium may be, for example, of the genus Methylobacterium. For example, the methylotrophic bacterium may be Methylobacterium organophilum.

The bacteria used in the composition of the present invention may be, for example, a consortium of a methanotrophic bacterium and a methylotrophic bacterium. The consortium may be, for example, a bacterium of the genus Methylocystis and a bacterium of the genus Methylobacterium.

The composition of the present invention may be used in various forms such as feed, a feed additive, food, or functional health food. In such cases, the composition may further comprise additional components suitable for use in such forms.

In addition, the present invention relates to a method for reducing methane emission from a ruminant animal.

The method of the present invention comprises a step of feeding the above-described composition for reducing methane emission to a ruminant animal.

The composition may be fed, for example, by mixing the composition with feed, or may be fed separately from feed.

The ruminant animal to be fed may be, for example, of the same species as the ruminant animal from which the methanotrophic bacterium or the methylotrophic bacterium is derived.

In addition, the present invention relates to a feed composition for a ruminant animal, comprising a methanotrophic bacterium.

The methanotrophic bacterium may be as exemplified above.

The feed composition of the present invention may further comprise a methylotrophic bacterium. The methylotrophic bacterium may be as exemplified above.

The ruminant animal to be fed may be, for example, of the same species as the ruminant animal from which the methanotrophic bacterium or the methylotrophic bacterium is derived.

The bacteria used in the composition of the present invention may be, for example, a consortium of a methanotrophic bacterium and a methylotrophic bacterium. The consortium may be, for example, a bacterium of the genus Methylocystis and a bacterium of the genus Methylobacterium.

The feed composition of the present invention can improve fermentation efficiency by removing methane, which is a by-product of the rumen fermentation process. In addition, the methanotroph metabolizes methane to provide an additional energy source (volatile fatty acids) and protein (methanotroph cells) to the ruminant animal, thereby improving feed efficiency. Furthermore, beneficial functional substances such as vitamins and carotenoids produced by the methanotrophs can also be supplied to the ruminant animal, so that the known efficacies/effects of such components can also be obtained.

The feed composition of the present invention may further comprise conventional ingredients included in feed for a ruminant animal.

In addition, the present invention relates to a feed additive composition for a ruminant animal, comprising a methanotrophic bacterium.

The methanotrophic bacterium may be as exemplified above.

The feed additive composition of the present invention may further comprise a methylotrophic bacterium. The methylotrophic bacterium may be as exemplified above.

The ruminant animal to be fed may be, for example, of the same species as the ruminant animal from which the methanotrophic bacterium or the methylotrophic bacterium is derived.

The bacteria used in the composition of the present invention may be, for example, a consortium of a methanotrophic bacterium and a methylotrophic bacterium. The consortium may be, for example, a bacterium of the genus Methylocystis and a bacterium of the genus Methylobacterium.

The feed additive composition of the present invention may be added to conventional feed for a ruminant animal. The amount of addition is not particularly limited, and may be, for example, 0.1 to 50 parts by weight, 0.1 to 30 parts by weight, or 0.1 to 10 parts by weight relative to 100 parts by weight of the feed.

The feed additive composition of the present invention can improve fermentation efficiency by removing methane, which is a by-product of the rumen fermentation process. In addition, the methanotroph metabolizes methane to provide an additional energy source (volatile fatty acids) and protein (methanotroph cells) to the ruminant animal, thereby improving feed efficiency. Furthermore, beneficial functional substances such as vitamins and carotenoids produced by the methanotrophs can also be supplied to the ruminant animal, so that the known efficacies/effects of such components can also be obtained.

Hereinafter, the present invention will be described in more detail by way of Examples.

### EXAMPLES

### Methods

### Enrichment and Isolation of Methanotrophic Consortium

After collecting a sample from inside the rumen of a Hanwoo cow, the sample was immediately immersed in sterile nitrate mineral salt medium (ATCC medium: 1306) supplemented with 10 mM CuCl2 (NMS-Cu), and then cultured for one week at 30°C in a serum bottle filled with a gas mixture of methane and air at a 20:80 ratio. After one week, the enrichment was diluted at a ratio of 1:10, placed in fresh NMS-Cu medium, and cultured for one more week under the same conditions as mentioned above. This procedure was repeated for 8 weeks to ensure enrichment of methanotrophs while reducing the possibility of growth of heterotrophs. To monitor the presence of methanotrophs, DNA was extracted weekly from the liquid culture throughout the entire enrichment period and screened using pmoA and methanotroph 16S rRNA specific primers (Table 1).

**[Table 1] Primers used to monitor the growth of methanotrophs during the enrichment process**

| **Primer Name** | **Target** | **Sequence** | **SEQ ID NO:** |
|---|---|---|---|
| MethT1dF | Methylomonas, Methylobacter, Methylomicrobium, Methylococcus | CCTTCGGGMGCYGACGAGT | 1 |
| MethT1bR | | GATTCYMTGSATGTCAAGG | 2 |
| 27F | Methylocystis, Methylosinus | AGAGTTTGATCMTGGCTCAG | 3 |
| MethT2R | | CATCTCTGRCSAYCATACCGG | 4 |
| Tlmob117F | Methylococcaceae (family) | GTAAYGCRTAGGAATCTGCC | 5 |
| Tlmob1144f | | CGGCAGTCTCCYTAGAGTTC | 6 |
| Tllmob445F | Methylocystaceae, Beijerinckaceae (family) | GGGAMGATAATGACGGTACCWGG | 7 |
| Tllmob1416F | | GCCTTCGGGTARARCCAACTCC | 8 |
| A189b | pmoA (not clear) | GGKGACTGGGACTTCTSG | 9 |
| mb661 | | CCGGMGCAACGTCYTTACC | 10 |
| mmox r1403 | sMMO | TGGCACTCGTAGCGCTCCGGCTC | 11 |
| mmox f901 | | TSAARACSTGGAACCGCTGGGT | 12 |

After 8 weeks, the liquid culture was spread onto NMS-Cu agar plates. To isolate methanotrophs, several colonies were picked and repeatedly subcultured by streaking. Finally, the colonies were transferred by spreading onto a 76 mm polycarbonate membrane filter with a 0.2 µm pore size (Sterlitech PCT027630). During spreading, the membrane was placed on an NMS-Cu agar plate serving as a support. The polycarbonate membrane filter was then immediately transferred onto a Petri dish containing 30 mL of fresh NMS-Cu medium so that the polycarbonate membrane floated on the medium. The dish was then placed at 30°C in a sealed chamber filled with a methane and air (50:50) mixture. The membrane was regularly observed, and the chamber was replaced with fresh methane-air mixture every 2 days. After 2 weeks of cultivation, pinkish colonies were observed. The colonies were then directly transferred to fresh liquid NMS medium and cultivated in a serum bottle at 30°C with shaking at 180 rpm for one week. The isolated colonies were named NC75PC, NC77PC, and NC52PC.

### Growth Characterization

The methanotroph growth experiments were performed in 120 mL serum bottles containing 30 mL of NMS-Cu medium. The bottles were sealed with butyl rubber stoppers and filled with a gas mixture of 20% (v/v) CH4 and 80% (v/v) air. The same methane:air mixture was used for all growth experiments. The three methanotroph isolates (NC52PC, NC75PC, NC77PC) were cultured in a shaking incubator at the rumen temperature of 39°C and a shaking speed of 180 rpm, and the growth rates were measured. Growth was monitored by absorbance (OD600) using an Ultrospec 10 cell density meter (Amersham Biosciences). Methane consumption during the cultivation process was analyzed using a gas chromatography flame ionization detector (GC-FID). All experiments were performed in triplicate.

### Morphological Characterization

Live cells of the methanotroph-methylotroph consortium NC52PC were observed using oil emulsion under a phase-contrast microscope at 400× magnification (Nikon 80i microscope from Japan equipped with a camera). To observe the precise morphology of the isolate by electron microscopy, samples were pretreated and observed using a scanning electron microscope (SEM) (Zeiss model EVO-MA-15 SEM).

### Genome Characterization

High-molecular-weight genomic DNA was extracted from NC52PC using Promega's Wizard^{®} HMW DNA Extraction Kit according to the manufacturer's instructions. Bacterial genome sequencing was performed by combining Oxford Nanopore Technologies long reads (ONT) and Illumina short reads sequencing technology (NovaSeq6000) to enhance accuracy and completeness. The assembled genome was annotated using Prokka version 1.14.6. Circular chromosomal maps of the two complete genomes and two circular plasmids were generated using the Proksee tool. To further confirm the taxonomic position, in silico DNA-DNA hybridization (isDDH), average nucleotide identity (ANI), and average amino acid identity (AAI) were calculated. The isDDH, ANI, and AAI values were also calculated for closely related species using the Type (Strain) Genome Server, the OrthoANIu algorithm, and the EzAAI tool of EZBioCloud.

### In Vitro Rumen Fermentation

Rumen fluid for in vitro rumen fermentation was collected from two Holstein-Friesian dairy cows that had undergone rumen cannulation surgery (body weight 874 ± 69 kg, 8 years old). The cows were fed twice daily with 60% concentrate and 40% bluegrass (Poa pratensis), with free access to clean drinking water and mineral blocks. Before the morning feeding, the rumen contents were collected in a thermos and immediately transported to the laboratory. The contents were then squeezed and filtered through four layers of surgical gauze and collected in an amber bottle. Thereafter, the gas inside the bottle was replaced with nitrogen for 30 minutes, and then the bottle was capped and stored while maintaining the temperature at 39°C. The filtered rumen fluid was then mixed with a buffer at a ratio of 1:3 (v/v) while maintaining an anaerobic environment. 30 mL of the buffered rumen fluid mixture was dispensed into 160 mL serum bottles under a flow of pure nitrogen gas. Each serum bottle contained 0.3 g of substrate composed of 80% bluegrass and 20% concentrate finely ground to pass through a 1 mm sieve, contained in a heat-sealed nylon bag. This in vitro fermentation consisted of two experimental sets, one set inoculated with 5×10⁷ CFU/mL of NC52PC and the control set not inoculated with NC52PC. Each set consisted of three replicates per time point at 0, 12, and 24 hours of cultivation. After mixing the buffered rumen fluid, substrate, and NC52PC inoculum (no inoculum in the control set) in the serum bottles, the internal gas was further replaced with nitrogen for 15 minutes, and then the bottles were sealed with butyl rubber caps and cultured in a shaking incubator at 100 rpm and 39°C.

During 0, 12, and 24 hours of cultivation, the total gas production from each bottle was recorded using the pressure transducer technique (Tagliapietra). Headspace gas (10 mL) was collected from each bottle using a syringe equipped with a two-way stopcock and then transferred into evacuated gas vials. Methane concentration was measured by gas chromatography with a flame ionization detector. After 0, 12, and 24 hours of cultivation, 10 mL liquid samples were collected and immediately frozen at -80°C for DNA extraction and microbial community analysis. The pH was measured using a pH meter (LaquaTwin, Horiba, UK). After the cultivation period was completed, the nylon bags containing residual feed were rinsed with cold tap water and dried in an oven at 80°C for 48 hours. After the nylon bags were dried, they were cooled to room temperature and weighed. Dry matter digestibility was calculated by subtracting the weight of the dried nylon bag from the initial weight.

### Animal Experimental Design

This study was conducted at the affiliated animal farm of Sunchon National University, and approval for all animal experiments was obtained from the university's Institutional Animal Care and Use Committee. Twelve Hanwoo steers (Bos taurus, 15 months old, initial body weight 448 ± 43 kg) were subjected to a 3×3 Latin square design with three replications, each lasting 28 days for a total experimental period of 84 days. The experimental design followed a cyclic pattern consisting of four phases: a 17-day feeding period, a 3-day data collection and analysis period, a 1-day sample collection and weighing period, and an 11-day washout period. During the washout period, the cattle were fed the same feed without feed supplementation to minimize carryover effects from residual microorganisms administered in the previous cycle. The animals were initially grouped by body weight and then randomly assigned to one of three treatments differing in feed supplement: control (CON), a mixture of 20 g of wheat bran and 20 mL of NMS-Cu medium without NC52PC bacteria; low-concentration NC52PC (LOW), a mixture of 20 g of wheat bran and 20 mL of NMS-Cu medium in which NC52PC was suspended at 3×10⁷ CFU/mL; and high-concentration NC52PC (HIGH), a mixture of 20 g of wheat bran and 20 mL of NMS-Cu medium in which NC52PC was suspended at 3×10⁸ CFU/mL. The experimental animals were fed a standard feed mixed with 8.5 kg of concentrate and 3.5 kg of forage (bluegrass) at a ratio of 70.8:29.2. Detailed nutritional composition is shown in Table 2. All treatment mixtures were prepared weekly and refrigerated, and then thoroughly mixed into the daily feed ration. Feeding was performed four times a day, and the supplement was provided at 09:00 AM.

**[Table 2] Nutritional composition of feed used in animal experiments**

| **Chemical composition (% of DM)** | **Value** |
|---|---|
| Moisture | 10.52 |
| Crude protein | 14.10 |
| Crude fat | 3.08 |
| Crude fiber | 7.37 |
| Total ash | 6.06 |
| Calcium | 1.40 |
| Potassium | 0.54 |
| Acid detergent fiber (ADF) | 12.53 |
| Neutral detergent fiber (NDF) | 26.41 |

### CH4 Emission Measurement

Enteric methane (CH4) emission was evaluated according to the Hristov experimental method, slightly modified to suit the experimental environment, using a GreenFeed (GF) device (C-Lock, Rapid City, SD, USA). Before starting the experiment, all experimental animals were trained to adapt to the GF device to minimize potential psychological stress. CH4 emission was monitored at 8 different intervals for 3 consecutive days during each measurement period. Measurements were conducted for 3 days at intervals of 0, 3, 6, 9, 12, 15, 18, and 21 hours based on the feeding time. The GF device was placed at one corner of a wide pen, and the experimental animals were sequentially moved from individual stalls to this experimental pen at each measurement interval. During measurement, molasses-coated concentrate pellets (250-300 g/session) were provided to induce the experimental animals to approach the GF device and assume the correct head-down posture within the hood. The amount of pellets ingested at this time was not included in the dry matter intake (DMI) calculation. All relevant data, including the entry and exit times of the animals at the GF device, standard gas calibration details, CO2 recovery timing, and gas emission measurements, were transmitted to C-Lock. CH4 production (g/d) data were calculated using a web-based data management system. CH4 yield (g/kg DMI) and CH4 intensity (g/d/kg BW^0.75) were also calculated.

### Rumen Fluid Sample Collection and Rectal Temperature Measurement

On day 16 of the animal experiment, before the morning feeding time, rumen fluid was collected from each cow using an oral tube. To prevent contamination by saliva, the first 300 mL of rumen fluid was discarded. Immediately after collection, rumen pH was measured using a pH meter (SevenCompactTM pH/Ion meter S220, Mettler Toledo, Greifensee, Switzerland). Thereafter, each rumen fluid sample was divided into 3 separate samples, placed on dry ice, and transported to the laboratory. They were then stored at -80°C for subsequent analysis of ammonia nitrogen (NH3-N), volatile fatty acids (VFA), rumen microorganisms, and the like. In addition, around 12:00 noon on the day of rumen fluid sample collection, the rectal temperature (RT) of the cattle was measured using a digital thermometer (WPT-1, CAS, Yangju, Korea).

### Analysis of Rumen NH3-N and Volatile Fatty Acid Concentrations

NH3-N concentration was measured using a Libra S22 spectrophotometer (CB40FJ, Biochrom, Cambourne, UK) according to the protocol developed by Chaney and Marbach. VFA concentration was measured using high-performance liquid chromatography (HPLC; Agilent Technologies 1200 series, Agilent Technologies, Waldbronn, Germany). To perform HPLC, a UV detector (set at 210 nm and 220 nm), a METACARB87H column (Varian, Palo Alto, CA, USA), and a buffered solvent (0.0085 N H2SO4; flow rate 0.6 mL/min) were used.

### Calculation and Statistical Analysis

All data on DMI, CH4 emission, rumen fermentation, and the rumen microbiome were analyzed using the MIXED procedure of SAS. Treatment, period, and animal were considered in the model, as appropriate. Differences among treatments were tested using an appropriate multiple comparison test. All analyses were performed using SAS (version 9.4, SAS Institute, Cary, NC, USA). Statistical significance was set at a p-value < 0.05.

### Results

### Isolation and Characterization of NC52PC

Methanotrophs are mainly isolated and cultured under aerobic environments, and have not previously been isolated from the rumen, which is known to be anaerobic, or cultured under rumen environmental conditions. In the present invention, aerobic methanotrophs were successfully isolated and cultured from rumen samples. This is because aerobic methanotrophs are highly likely to also be present in the rumen due to oxygen entering the rumen through the rumen epithelium. Through serial subculture and repeated streaking on agar plates, colonies were isolated and again transferred onto polycarbonate membranes to minimize contamination by heterotrophs that can be found on agar plates.

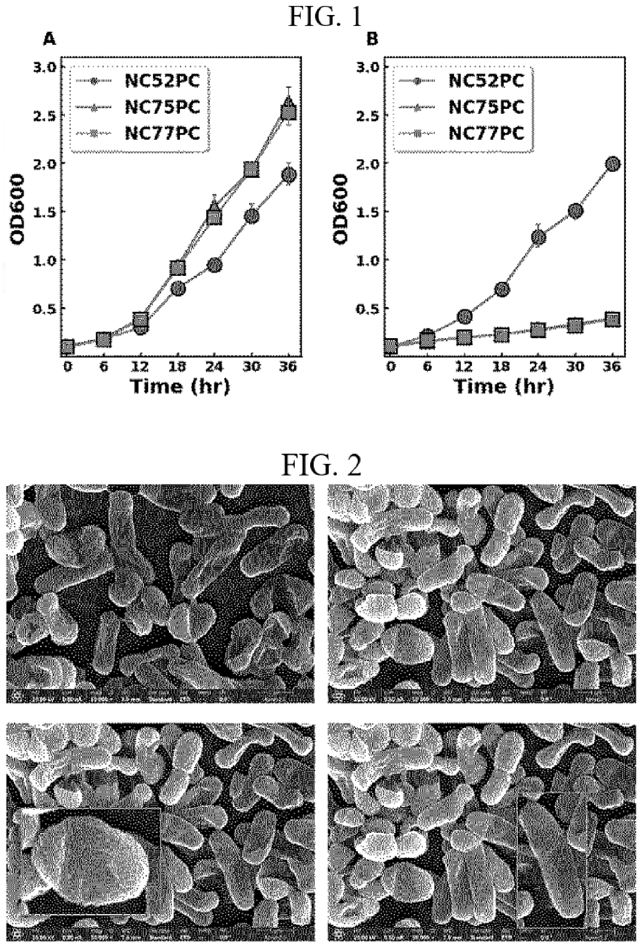

The growth of three promising isolates (NC75PC, NC77PC, NC52PC) obtained on the polycarbonate membrane was tested at 30°C and at the rumen temperature of 39°C. The isolates NC75PC, NC77PC, and NC52PC grew at 30°C for 36 hours with specific growth rates of 0.1164 h⁻¹, 0.1172 h⁻¹, and 0.0915 h⁻¹, respectively, reaching OD600 values of 2 or higher (FIG. 1). However, when cultured at 39°C, the growth rates of isolates NC75PC and NC77PC were significantly reduced to 0.0308 h⁻¹ and 0.0275 h⁻¹, respectively. In contrast, NC52PC grew robustly even at 39°C, exhibiting a specific growth rate of 0.1075 h⁻¹, similar to that at 30°C, and was therefore used in the subsequent in vitro and in vivo rumen fermentation experiments.

**[Table 3] Growth rates of three rumen isolates**

| **Isolate** | **Temperature (°C)** | **µmax (h⁻¹)** | **Td (h)** |
|---|---|---|---|
| NC52PC | 30 | 0.0915 ± 0.0030 | 7.6 ± 0.25 |
| | 39 | 0.1075 ± 0.0094 | 6.5 ± 0.58 |
| NC75PC | 30 | 0.1164 ± 0.0023 | 6.0 ± 0.12 |
| | 39 | 0.0308 ± 0.0016 | 22.6 ± 1.18 |
| NC77PC | 30 | 0.1172 ± 0.0014 | 5.9 ± 0.07 |
| | 39 | 0.0275 ± 0.0046 | 25.7 ± 4.50 |

Data are expressed as mean ± standard deviation (SD).

**SEM:** Examination of NC52PC by scanning electron microscopy revealed two morphologically distinct cell types (FIG. 2). One type of cell appeared as rod-shaped bacilli with a smooth surface of approximately 2.4~2.9 × 0.8~1 microns in size. The second type appeared as curved cocci with a rough surface of approximately 1.3~1.5 × 0.8~1 microns in size. In addition, genome sequencing revealed two genomic DNAs, one closely related to the methanotroph Methylocystis echinoides and the other found to be similar to the methylotroph Methylobacterium organophilum. Therefore, the rod-shaped bacilli with a smooth surface were presumed to be Methylobacterium sp., and the other curved cocci with a rough surface were presumed to be Methylocystis sp.

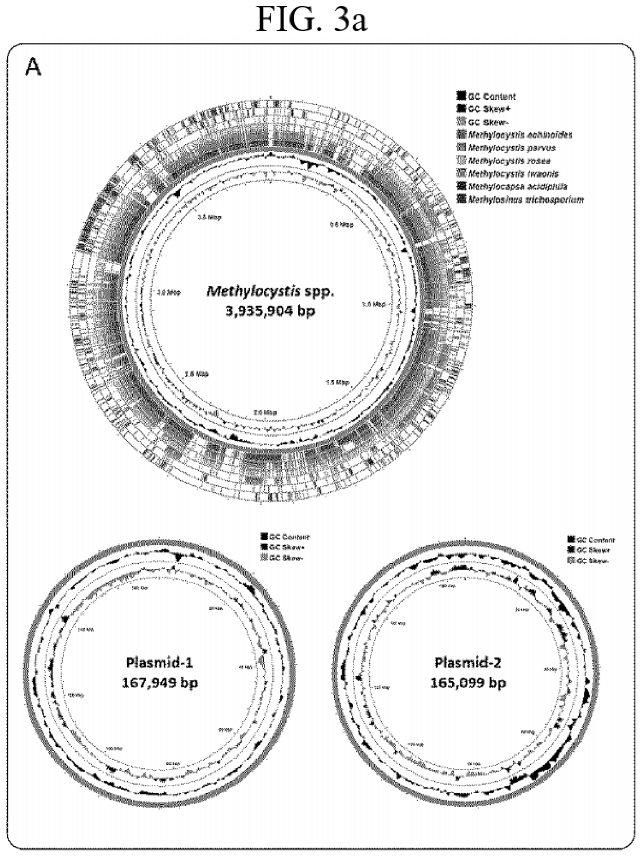

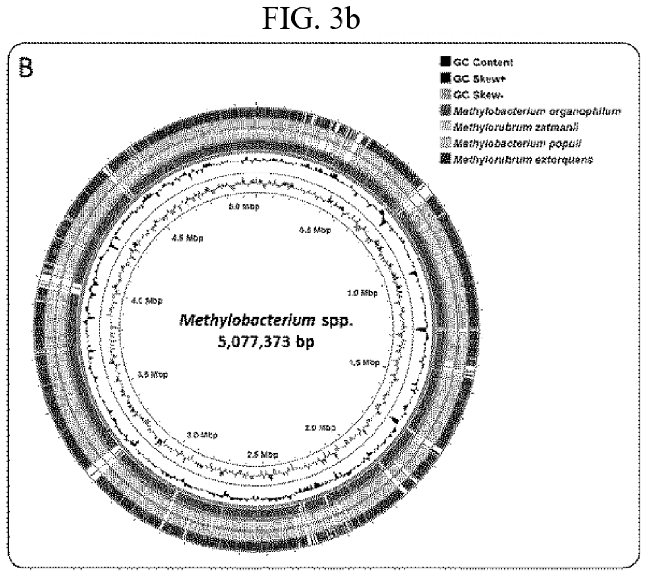

**Genome Analysis:** The genome of isolate NC52PC was analyzed using hybrid long-read and short-read sequencing. Sequencing analysis yielded a total of four contigs consisting of two circular chromosomes and two circular plasmids. According to BlastX analysis, the larger chromosome of 5.1 Mbp showed similarity with the genus Methylobacterium. The other chromosome of 3.95 Mbp and the two plasmids belonged to the genus Methylocystis. The sizes of the two plasmids were 167 kb and 165 kb. Genome features such as GC content, tRNAs, rRNA, and the number of genes and proteins were calculated using Prokka (Table 4). The visualization of the genome of each species of NC52PC and closely related species is shown in FIG. 3.

**[Table 4] Genome features of the two circular chromosomes of NC52PC**

| **Traits** | **Methylobacterium sp.** | **Methylocystis sp.** |
|---|---|---|
| Genome size | 5.07 Mbp | 3.9 Mbp |
| Contigs | 1 | 3 |
| Circular | Yes | Yes |
| GC% | 69.98 | 64.46 |
| tRNA | 150 | 150 |
| rRNA | 24 | 12 |
| CDS | 4683 | 3884 |
| 16S | 4 | 2 |
| pmoAs | - | 2 |
| Integrated plasmids | - | 2 |
| GenBank Accession No. | CP168955 | CP170127 |

A genome-based comparison of Methylocystis sp. and Methylobacterium sp. present in NC52PC was performed with closely related species, and average nucleotide identity (ANI), in silico DNA-DNA hybridization (DDH), and average amino acid identity (AAI) were calculated. The ANI, AAI, and DDH values between the Methylocystis isolate and its closest relative Methylocystis echinoides LMG27198 were 81.1%, 81.4%, and 25.8%, respectively, all of which were lower than the threshold values (95% for ANI or AAI, 70% for DDH) (Table 5). Therefore, it is suggested that the Methylocystis strain of NC52PC represents a new species of the genus Methylocystis belonging to the family Methylocystaceae. Likewise, comparison of the genome of Methylobacterium sp. in NC52PC with closely related Methylobacterium species showed the highest similarity with Methylobacterium organophilum WPA_B, with ANI, AAI, and DDH values of 98.6%, 98.8%, and 88.5%, respectively (Table 5). Therefore, the Methylobacterium sp. of the NC52PC consortium is most likely Methylobacterium organophilum. Further isolation of the methanotroph alone from NC52PC, which is a consortium of a methanotroph and a methylotroph, was technically difficult, and the growth rate of the isolated methanotroph was severely impaired. To maximize methane reduction, it is ideal to use a methanotroph having a high growth rate that can rapidly metabolize methane. Therefore, for methane reduction in rumen fermentation, it may be best to utilize a consortium of a methanotroph and a methylotroph, rather than a pure methanotroph. The methylotroph can metabolize excess methanol generated during the methane oxidation process to reduce methanol toxicity and promote the growth of the methanotroph. In addition, the exchange of essential nutrients between Methylocystis and Methylobacterium organophilum may promote the overall growth performance of this consortium.

**[Table 5] Genome comparison of Methylocystis sp. and Methylobacterium sp. of NC52PC with other closely related species (isDDH: in silico DNA-DNA hybridization; ANI: average nucleotide identity; AAI: average amino acid identity).**

| **Rumen isolate NC52PC** | **Closely related members** | **isDDH(%)** | **ANI(%)** | **AAI(%)** |
|---|---|---|---|---|
| Methylobacterium spp. | Methylobacterium organophilum WPA_B | 88.5 (87.6-90.3) | 98.59 | 98.79 |
| | Methylobacterium populi BJ001 | 26.6 (25.4-28.0) | 81.68 | 78.16 |
| | Methylorubrum extorquens AM1 | 24.5 (23.9-25.1) | 80.77 | 77.58 |
| | Methylorubrum zatmanii LMG 6087 | 28.2 (27.7-29.2) | 80.75 | 77.48 |
| Methylocystis spp. | Methylocystis echinoides LMG 27198 | 25.76 (24.6-27.2) | 81.07 | 81.35 |
| | Methylocystis iwaonis JCM 34278T | 23.66 (22.8-24.4) | 80.39 | 80.42 |
| | Methylocystis parvus OBbp | 23.9 (23.4-24.7) | 80.02 | 80.46 |
| | Methylocystis rosea SV98 | 19.2 (17.8-21.9) | 77.22 | 74.67 |
| | Methylosinus trichosporium OB3b | 18.5 (15.8-21.8) | 75.97 | 69.36 |

**[Table 6] GenBank accession information for NC52PC**

| **SUBID** | **BioProject** | **BioSample** | **Localid** | **Accession** | **Organism** |
|---|---|---|---|---|---|
| SUB14691566 | PRJNA1155250 | SAMN43453749 | Contig3C | CP170125 (plasmid 1) | Methylocystis sp. NC52PC |
| SUB14691566 | PRJNA1155250 | SAMN43453749 | Contig4 | CP170126 (plasmid 2) | Methylocystis sp. NC52PC |
| SUB14691566 | PRJNA1155250 | SAMN43453749 | Contig2C | CP170127 (Genome) | Methylocystis sp. NC52PC |
| SUB14700942 | PRJNA1155264 | SAMN43454258 | - | CP168955 (genome) | Methylobacterium organophilum NC52PC |

### In Vitro Rumen Fermentation Parameters

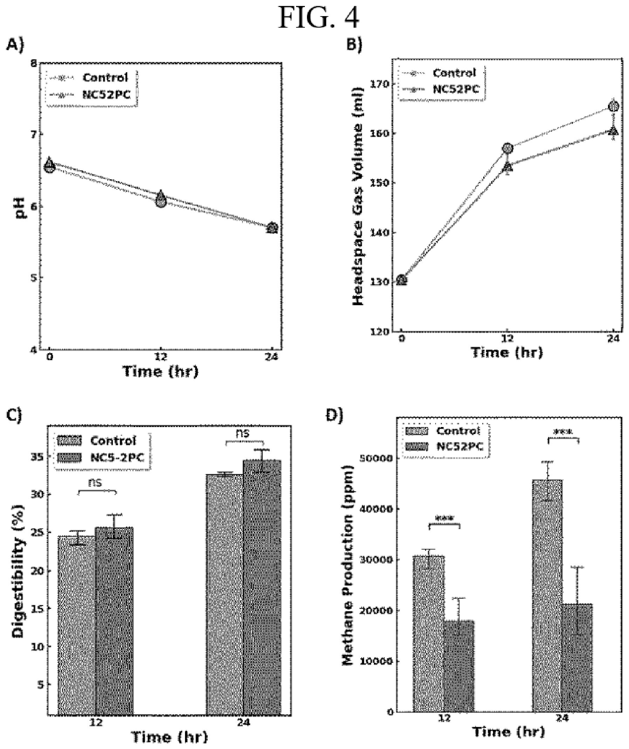

During in vitro rumen fermentation, no significant difference was observed in pH, total gas production, or digestibility between the control and NC52PC-inoculated samples at any sampling point, except for methane production (p-value > 0.05). After 24 hours, the total headspace volume of the serum bottles increased from 130 mL to more than 160 mL. Dry matter degradation continuously increased from 25% at 12 hours to more than 32% at 24 hours, indicating that rumen fermentation was actively proceeding during the experimental period. In addition, various organic acids were synthesized during the rumen fermentation process, and the pH levels in both samples decreased from 6.5 to 5.8. When NC52PC was inoculated, methane production was significantly reduced by 41.7% and 53.6% at 12 hours and 24 hours, respectively (p-value < 0.01) (FIG. 4). The slow rate of increase in methane production from 12 to 24 hours in the NC52PC-inoculated group indicates sustained methanotrophic activity. Methane consumption may have been sustained because the population of Methylocystis remained relatively stable for 24 hours. Although NC52PC fundamentally requires oxygen for growth and methane oxidation, in order to confirm whether NC52PC has the ability to oxidize methane and grow even under anoxic conditions, methane consumption was tested using 20% methane under anoxic conditions in NMS-Cu medium supplemented with 0.60 g/L cysteine. As a result, it was shown that the methane concentration decreased from 180,000 ppm to 168,000 ppm and 160,000 ppm after 24 hours and 48 hours, respectively. That is, NC52PC was able to consume approximately 20,000 ppm of methane over 48 hours even without oxygen. This highlights the versatile applicability of the aerobic methanotroph NC52PC and its ability to stably oxidize methane despite an insufficient supply of oxygen. Methane oxidation by aerobic methanotrophs in an anaerobic environment is possible by utilizing other alternative electron acceptors available in the rumen contents. It has been reported that microorganisms belonging to the families Methylomonadaceae and Methylocystaceae can perform nitrate/nitrite or mineral oxidedependent methane oxidation under oxygen limitation. NC52PC may have evolved to utilize denitrification or mineral reduction processes in oxygen-free environments such as the rumen.

### Effect of NC52PC on the In Vitro Rumen Microbial Composition

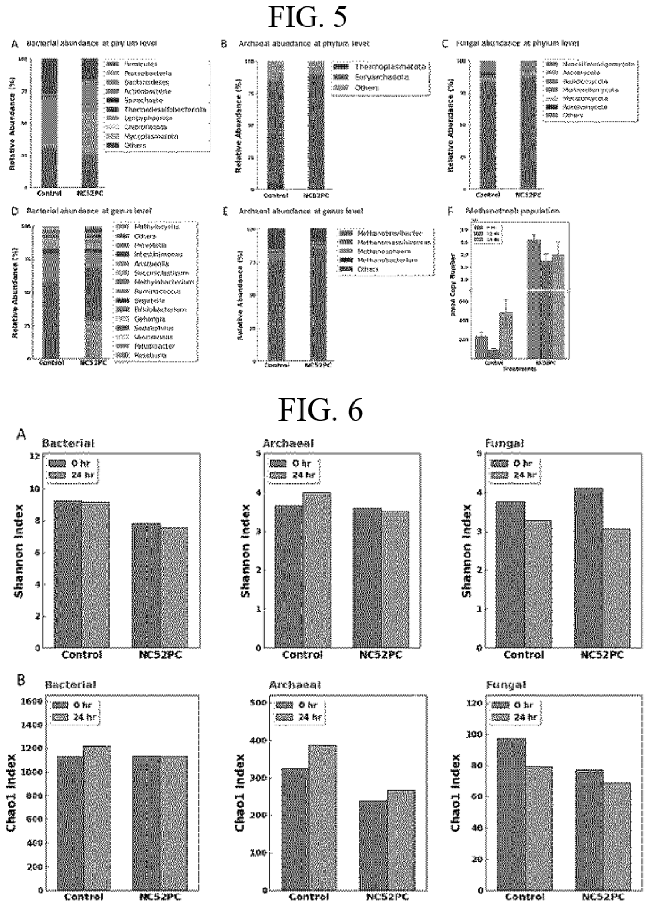

Total genomic DNA was extracted from both the control and NC52PC samples from 3 technical replicates of the 24-hour samples and 1 replicate of the 0-hour samples. To confirm differences in the composition, abundance, and diversity of rumen microorganisms between the control and NC52PC samples after 24 hours of in vitro fermentation, 16S rRNA (V3-V4) gene sequencing analysis was performed. Overall, a total of 18 bacterial phyla and 276 bacterial genera were detected in the integrated experimental samples. Bacteroidetes, Firmicutes, Proteobacteria, and Actinobacteria were the dominant phyla, accounting for up to 80% of the total bacterial ASVs (FIG. 5). In the control, Prevotella was the most dominant genus (16.56%), followed by Intestinimonas (4.21%), Aristaeella (4.20%), Succiniclasticum (2.51%), Ruminococcus (2.41%), Sodaliphilus (2.17%), Lentimicrobium (1.63%), Bifidobacterium (1.41%), Gehongia (1.39%), and Paludibacterium (1.32%) as dominant. Meanwhile, in the NC52PC-inoculated samples, the most dominant genus was Methylocystis (28.7%), followed by Prevotella (9.5%), Aristaeella (3.2%), Sodaliphilus (3.2%), Intestinimonas (3.16%), Methylobacterium (2.2%), Ruminococcus (1.74%), Segatella (1.61%), Succiniclasticum (1.16%), and Bifidobacterium (1.1%). Because Methylocystis was the dominant genus in the NC52PC samples, the relative abundance of other dominant bacterial genera such as Prevotella, Aristaeella, Intestinimonas, Ruminococcus, and Lentimicrobium was significantly lower (p-value < 0.05).

In both the control and NC52PC 24-hour samples, the archaeal community was mostly dominated by the genus Methanobrevibacter (control 78%, NC52PC 86%), followed by Methanomassiliicoccus and Methanosphaera. In the case of Methanobrevibacter, which is a dominant methanogenic archaeon in the rumen, its number relatively increased after 24 hours, so the addition of NC52PC did not have a negative effect on the methanogenic community. Regarding the fungal community, the three phyla Neocallimastigomycota, Ascomycota, and Basidiomycota dominated in both the control and NC52PC samples, accounting for more than 80% of the total fungal ASVs (FIG. 5). In particular, Neocallimastigomycota, which expresses various enzymes involved in lignocellulose degradation, slightly increased, which can promote feed digestion.

As a result of analyzing alpha diversity, in the 24-hour samples, bacterial species diversity (Shannon index) was significantly higher in the control samples than in NC52PC (p-value < 0.05), but it was found that there was no significant effect on species richness (Chao 1 index). In addition, although there was no effect on archaeal diversity, the Chao 1 index was significantly lower in NC52PC than in the control samples (p-value < 0.05), thereby having a significant effect on archaeal species richness. In addition, differences in fungal species diversity and richness were not significant (p-value > 0.05).

### In Vivo Rumen Fermentation

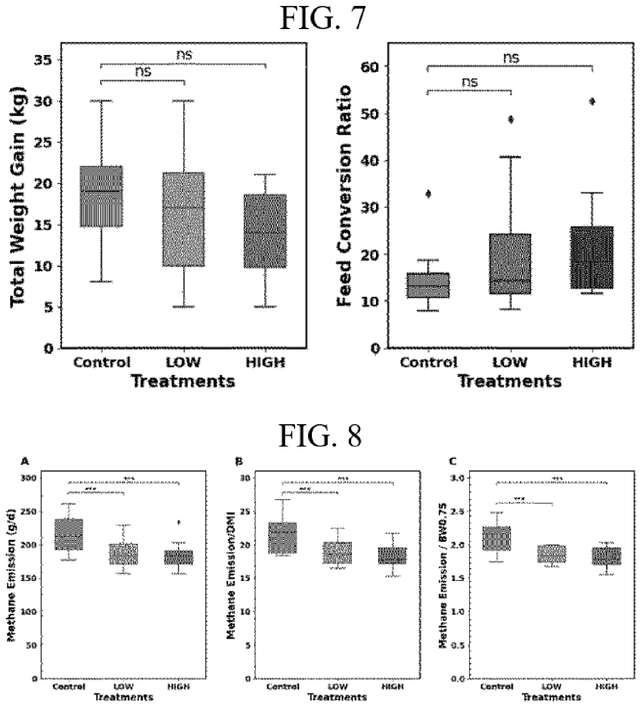

Overall, DMI (kg/d), total body weight gain (kg), and feed conversion ratio (FCR) did not differ significantly between treatment groups (p > 0.05) (FIG. 7). The rumen fermentation characteristics of CON, LOW, and HIGH are shown in Table 7. Butyrate and total volatile fatty acid (VFA) production of CON were significantly lower than those of the LOW and HIGH treatments. As a result, the rumen pH of NC52PC-treated LOW (6.30) and HIGH (6.29) may have been lower than that of CON (6.55). Both LOW and HIGH did not significantly affect other fermentation characteristics, including ammonia content, acetate, propionate, and the A:P ratio. Most importantly, CH4 emission (g/d), CH4 yield (g/kg DMI), and CH4 intensity (g/kg BW0.75) were significantly higher in the control than in the LOW and HIGH NC52PC-treated cattle (p < 0.05) (FIG. 8), while the total CO2 levels remained similar among all treated cattle (data not shown).

**[Table 7] Effects of methanotroph-based probiotics on pH, ammonia-nitrogen, and volatile fatty acid production**

| **Item** | **Treatments¹** | | | **SEM²** | **P-value** |
|---|---|---|---|---|---|
| | **CON** | **LOW** | **HIGH** | | |
| pH | 6.55 ± 0.22 | 6.30 ± 0.23 | 6.29 ± 0.24 | 0.067 | 0.0191 |
| NH3-N (mg/dL) | 3.89 ± 2.53 | 3.65 ± 2.14 | 3.32 ± 2.32 | 0.673 | 0.9163 |
| Acetate (mmol/L) | 57.93 ± 10.68 | 62.35 ± 7.66 | 65.75 ± 8.05 | 2.539 | 0.1567 |
| Propionate (mmol/L) | 18.74 ± 4.65 | 22.10 ± 6.21 | 22.48 ± 5.07 | 1.583 | 0.2934 |
| Butyrate (mmol/L) | 34.70 ± 20.20 | 41.13 ± 19.03 | 49.32 ± 29.52 | 6.616 | 0.0031 |
| Total VFA (mmol/L) | 111.37 ± 33.12 | 125.88 ± 22.14 | 137.55 ± 33.04 | 8.496 | 0.0050 |
| A:P³ | 3.17 ± 0.59 | 2.93 ± 0.44 | 3.03 ± 0.61 | 0.157 | 0.7796 |

| | | | | | |
|---|---|---|---|---|---|
| Data are expressed as mean ± standard deviation (SD). ¹Treatments: CON, basal diet (0.2% wheat bran); LOW, basal diet + 0.2% methanotroph (3×10⁷ CFU/mL); HIGH, basal diet + 0.2% methanotroph (3×10⁸ CFU/mL). ²SEM, standard error of the mean. ³A:P, acetate to propionate ratio. | | | | | |

### Conclusion

The study results showed that when methanotroph-based probiotics at 3×10^8 CFU/mL were administered to 12 Hanwoo cattle for 17 days, methane emission was successfully reduced by more than 14% without adversely affecting overall animal health. Methanotroph-based probiotics have tremendous potential to mitigate methane emission from ruminant animals and can be used as a promising feed additive to combat climate change.

## Claims

1. A composition for reducing methane emission from a ruminant animal, comprising a methanotrophic bacterium.

2. The composition for reducing methane emission from a ruminant animal according to claim 1, wherein the methanotrophic bacterium is derived from the rumen of a ruminant animal.

3. The composition for reducing methane emission from a ruminant animal according to claim 1, wherein the methanotrophic bacterium is a bacterium of the genus Methylocystis, the genus Methylomonas, the genus Methylococcus, the genus Methylosinus, or the genus Methylomicrobium.

4. The composition for reducing methane emission from a ruminant animal according to claim 1, further comprising a methylotrophic bacterium.

5. The composition for reducing methane emission from a ruminant animal according to claim 4, wherein the methylotrophic bacterium is derived from the rumen of a ruminant animal.

6. The composition for reducing methane emission from a ruminant animal according to claim 4, wherein the methylotrophic bacterium is a bacterium of the genus Methylobacterium.

7. The composition for reducing methane emission from a ruminant animal according to claim 4, wherein the methylotrophic bacterium is Methylobacterium organophilum.

8. The composition for reducing methane emission from a ruminant animal according to claim 1, wherein the ruminant animal is a cow, a giraffe, a deer, a sheep, or a camel.

9. A method for reducing methane emission from a ruminant animal, comprising feeding the composition according to any one of claims 1 to 8 to a ruminant animal.

10. A feed composition for a ruminant animal, comprising a methanotrophic bacterium.

11. The feed composition for a ruminant animal according to claim 10, wherein the methanotrophic bacterium is derived from the rumen of a ruminant animal.

12. The feed composition for a ruminant animal according to claim 10, wherein the methanotrophic bacterium is a bacterium of the genus Methylocystis, the genus Methylomonas, the genus Methylococcus, the genus Methylosinus, or the genus Methylomicrobium.

13. The feed composition for a ruminant animal according to claim 10, further comprising a methylotrophic bacterium.

14. The feed composition for a ruminant animal according to claim 13, wherein the methylotrophic bacterium is derived from the rumen of a ruminant animal.

15. The feed composition for a ruminant animal according to claim 13, wherein the methylotrophic bacterium is a bacterium of the genus Methylobacterium.

16. The feed composition for a ruminant animal according to claim 13, wherein the methylotrophic bacterium is Methylobacterium organophilum.

17. A feed additive composition for a ruminant animal, comprising a methanotrophic bacterium.

18. The feed additive composition for a ruminant animal according to claim 17, wherein the methanotrophic bacterium is derived from the rumen of a ruminant animal.

19. The feed additive composition for a ruminant animal according to claim 17, wherein the methanotrophic bacterium is a bacterium of the genus Methylocystis, the genus Methylomonas, the genus Methylococcus, the genus Methylosinus, or the genus Methylomicrobium.

20. The feed additive composition for a ruminant animal according to claim 17, further comprising a methylotrophic bacterium.

21. The feed additive composition for a ruminant animal according to claim 20, wherein the methylotrophic bacterium is derived from the rumen of a ruminant animal.

22. The feed additive composition for a ruminant animal according to claim 20, wherein the methylotrophic bacterium is a bacterium of the genus Methylobacterium.

23. The feed additive composition for a ruminant animal according to claim 20, wherein the methylotrophic bacterium is Methylobacterium organophilum.
